(19) Europäisches Patentamt
European Patent Office
Office européen des brevets

(11) **EP 3 597 732 A1**

(12) **EUROPEAN PATENT APPLICATION**
published in accordance with Art. 153(4) EPC

(43) Date of publication:
**22.01.2020 Bulletin 2020/04**

(21) Application number: **18767265.4**

(22) Date of filing: **16.03.2018**

(51) Int Cl.:
*C12N 5/078* (2010.01)    *C12M 1/00* (2006.01)

(86) International application number:
**PCT/JP2018/010507**

(87) International publication number:
**WO 2018/169060 (20.09.2018 Gazette 2018/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.03.2017 JP 2017050824**

(71) Applicant: FUJIFILM Corporation
**Tokyo 106-8620 (JP)**

(72) Inventors:
• **TAKEI Toshiki**
 **Ashigarakami-gun**
 **Kanagawa 258-8577 (JP)**
• **YAMADA Tadanori**
 **Ashigarakami-gun**
 **Kanagawa 258-8577 (JP)**
• **HATA Kenichiro**
 **Ashigarakami-gun**
 **Kanagawa 258-8577 (JP)**

(74) Representative: **Hoffmann Eitle**
 **Patent- und Rechtsanwälte PartmbB**
 **Arabellastraße 30**
 **81925 München (DE)**

(54) **METHOD FOR SEPARATING MEGAKARYOCYTES FROM PLATELETS, AND PLATELET SEPARATION KIT**

(57)     An object of the present invention is to provide a method for efficiently separating megakaryocytes and platelets produced from the megakaryocytes, and a platelet separation kit for efficiently separating megakaryocytes and platelets produced from the megakaryocytes. According to the present invention, a method for separating megakaryocytes and platelets, including an incorporation step of incorporating magnetic fine particles into at least megakaryocytes; a culture step of culturing the megakaryocytes in a culture solution to produce platelets before and/or after the incorporation step; a magnetic field application step of applying a magnetic field to the culture solution after the incorporation step and the culture step; and a recovery step of recovering the culture solution after the magnetic field application step, is provided.

FIG. 1

RESOVIST-ADDED GROUP     RESOVIST-NON-ADDED GROUP

20 μm     20 μm

**Description**

**BACKGROUND OF THE INVENTION**

1. Field of the Invention

The present invention relates to a method for separating megakaryocytes and platelets produced from the megakaryocytes. The present invention further relates to a platelet separation kit.

2. Description of the Related Art

Platelets are used in blood transfusion therapy as a platelet formulation. A platelet formulation is generally prepared from blood obtained by blood donation, but a supply amount of platelet formulations is likely to be influenced by external factors such as a reduction in blood donors. Accordingly, attention has been focused on the development of platelet sources that can replace blood donations. In recent years, a technology has been reported for mass production of platelets in vitro by culturing megakaryocytes with use of pluripotent stem cells, hematopoietic precursor cells, and mesenchymal lineage cells as sources (Patent Document 1). Platelets are produced by breaking up the cytoplasm of megakaryocytes, and therefore a culture solution after platelet production contains a large number of megakaryocytes. There is a possibility that a platelet formulation has immunogenicity due to inclusion of megakaryocytes. Therefore, it is necessary to develop a technology for separating megakaryocytes and platelets produced from the megakaryocytes.

As a method for separating cells, a centrifugation method for separating cells based on a difference in specific gravity of cells is generally used. For example, Patent Document 2 discloses a platelet collection device including a centrifuge that has, in the inside thereof, a blood storage space communicating with an inlet port and an outlet port; a first line that is connected to the inlet port of the centrifuge and a blood collection means; a second line that is connected to the outlet port of the centrifuge; a plasma collection bag that has a first tube connected in the middle of the first line and a second tube connected to the second line; a platelet collection circuit that has a platelet collection bag connected to the second line; and a blood feeding pump that is provided in the first line, in which the blood collected by the centrifuge is separated into a plurality of blood components, and at least platelets of the separated blood components are collected in the platelet bag.

In addition, there are known methods in which, by mixing magnetic beads to which an antibody is bound and a cell culture solution, only cells having a specific marker are adsorbed to the magnetic beads, and a magnetic field applied, and therefore the cells are separated. For example, Patent Document 3 discloses a cell separation device characterized by including a micro-mixer that has a first fluid dividing means for dividing a fluid containing magnetic beads and predetermined cells into left and right, a second fluid dividing means for dividing the fluid divided left and right upward, a third fluid dividing means for dividing the fluid divided left and right downward, and a fluid combining means for combining the fluid divided upward and the fluid divided downward; and including a separator for separating the magnetic beads in the fluid and cells attached to the magnetic beads from the remainder of the fluid after the fluid permeated through the micromixer.

Prior Art Document

Patent Documents

Patent Document 1: WO09/122747A

Patent Document 2: JP2003-093499A

Patent Document 3: JP2006-006166A

**SUMMARY OF THE INVENTION**

In the case of separating platelets by a centrifugation method which separates cells based on a difference in specific gravity of cells as described in Patent Document 2, because specific gravities of megakaryocytes and platelets are close, a large centrifugal acceleration is required in a case of separating secretions thereof by the centrifugation method, and therefore there is a concern about activation and deformation of the secretions due to centrifugal force. In addition, megakaryocytes and platelets are considered to have no difference in the presence or absence of expression of membrane surface markers, and therefore a method for separating cells by adsorbing only cells having a specific marker to magnetic beads and applying a magnetic field as described in Patent Document 3 cannot be applied for separation of megakaryocytes and platelets.

An object of the present invention is to provide a method for efficiently separating megakaryocytes and platelets produced from the megakaryocytes, and a platelet separation kit for efficiently separating megakaryocytes and platelets produced from the megakaryocytes.

As a result of intensive studies to solve the above-described problems, the inventors of the present invention have found that megakaryocytes and platelets can be separated by introducing magnetic particles into the cytoplasm to magnetize the cells and then applying a magnetic field, and therefore have completed the present invention.

In other words, according to the present invention, the following invention is provided.

[1] A method for separating megakaryocytes and platelets, the method comprising:

an incorporation step of incorporating magnetic fine particles into at least megakaryocytes;
a culture step of culturing the megakaryocytes in a culture solution to produce platelets before and/or after the incorporation step;
a magnetic field application step of applying a magnetic field to the culture solution after the incorporation step and the culture step; and
a recovery step of recovering the culture solution after the magnetic field application step.

[2] The method according to [1], in which the megakaryocytes are cultured in the culture solution to produce the platelets, the magnetic fine particles are incorporated into the megakaryocytes and the platelets, a magnetic field is applied to the culture solution, and then the culture solution is recovered.

[3] The method according to [1], in which the magnetic fine particles are incorporated into the megakaryocytes, the megakaryocytes are cultured in the culture solution to produce platelets, a magnetic field is applied to the culture solution, and then the culture solution is recovered.

[4] The method according to any one of [1] to [3], in which the magnetic fine particles are fine particles having an average particle diameter of 10 to 1000 nm.

[5] The method according to any one of [1] to [4], in which the magnetic fine particles are superparamagnetic iron oxide particles.

[6] The method according to any one of [1] to [5], in which 1 to 1000 $\mu$g/ml concentration of the magnetic fine particles are added to the culture solution in the incorporation step.

[7] The method according to any one of [1] to [6], in which 10 to 400 mT of a magnetic field is applied to the culture solution in the magnetic field application step.

[8] A platelet separation kit for separating megakaryocytes and platelets, the kit comprising, at least: a culture vessel; magnetic fine particles; and magnetic field application means.

According to the method and the kit of the present invention, megakaryocytes and platelets produced from the megakaryocytes can be efficiently separated.

## BRIEF DESCRIPTION OF THE DRAWINGS

Fig. 1 shows results of Berlin blue staining of a cell section into which magnetic fine particles have been introduced.
Fig. 2 shows a state of a case where a cell into which magnetic fine particles have been introduced is not in contact with a magnet, and a case where the cell is in contact with the magnet.

## DESCRIPTION OF THE PREFERRED EMBODIMENTS

Hereinafter, the embodiment of the present invention will be described in detail.

A method for separating megakaryocytes and platelets according to the embodiment of the present invention, includes

an incorporation step of incorporating magnetic fine particles into at least megakaryocytes;
a culture step of culturing the megakaryocytes in a culture solution to produce platelets before and/or after the incorporation step;
a magnetic field application step of applying a magnetic field to the culture solution after the incorporation step and the culture step; and

a recovery step of recovering the culture solution after the magnetic field application step.

In manufacturing a platelet formulation used for blood transfusion therapy and the like, the method of the embodiment of the present invention can be used as a method for separating platelets in a case where a large amount of platelets are produced in vitro.

<Regarding megakaryocytes and platelets>

Megakaryocytes are cells derived from hematopoietic stem cells, and are cells having a polymorphonuclear nucleus formed via megakaryocyte precursor cells, megakaryoblasts, and promegakaryocytes. Megakaryocytes are present in the bone marrow and are the largest hematopoietic lineage cells having a diameter of 35 to 160 $\mu$m in the bone marrow. Megakaryocytes produce platelets. Thousands of platelets are produced from per megakaryocyte. After the cytoplasm of megakaryocytes is degraded to become platelets, the megakaryocytes lose their cytoplasm and become naked nuclei, and the naked nuclei are degraded by macrophages. Megakaryocytes referred to in the embodiment of the present invention may include, megakaryocyte precursors, megakaryoblasts, promegakaryocytes, and the like, in addition to mature megakaryocytes.

Platelets are a type of cellular component contained in blood. Platelets play a central role in thrombus formation, and have the action of hemostasis by platelet aggregation in a case where the vascular wall is damaged.

Megakaryocytes and platelets may be megakaryocytes and platelets collected from adult tissues, may be megakaryocytes and platelets differentiated from cells having a differentiation capacity, such as pluripotent stem cells, hematopoietic precursor cells, and mesenchymal lineage cells, may be megakaryocytes and platelets produced by using a direct reprogramming technique for cells that do not have a capacity to be differentiated into megakaryocytes by general methods, or may be a combination of the above megakaryocytes and platelets.

An organism species from which megakaryocytes and platelets are derived is not particularly limited, but is preferably mammals (for example, humans, mice, rats, hamsters, guinea pigs, sheep, pigs, monkeys, and the like), and is more preferably humans.

Examples of pluripotent stem cells include embryonic stem cells (ES cells), nuclear transfer embryonic stem cells (ntES cells), induced pluripotent stem cell (iPS cells), and the like, but are not limited thereto.

Examples of hematopoietic precursor cells include cells derived from bone marrow, cells derived from umbilical cord blood, cells derived from (G-CSF)-mobilized peripheral blood, middle lobe lung cells derived from ES cells, cells derived from peripheral blood, and the like, but are not limited thereto. Examples of these hematopoietic precursor cells include cells positive to CD34 (for example, CD34+ cells, CD133+ cells, SP cells, CD34+ CD38-cells, c-kit+ cells, or CD3-, CD4-, CD8-, and CD34+ cells) (WO2004/110139A).

Examples of mesenchymal lineage cells include mesenchymal lineage stem cells, adipocyte precursor cells, and the like, but are not limited thereto.

Examples of cells that do not have a capacity to be differentiated into megakaryocytes by general methods include fibroblasts and the like, but are not limited thereto.

It is sufficient that a method for producing megakaryocytes and platelets by differentiating cells having a differentiation capacity, such as pluripotent stem cells, hematopoietic precursor cells, and mesenchymal lineage cells is performed according to methods generally known to those skilled in the art, and the method is not particularly limited. By culturing cells having a differentiation capacity under appropriate culture conditions by using a differentiation-inducing medium suitable for differentiating the cells into megakaryocytes, it is possible to differentiate the cells having a differentiation capacity into megakaryocytes, and platelets are produced from the megakaryocytes.

As a method for producing megakaryocytes and platelets by using a direct reprogramming technique for cells that do not have a capacity to be differentiated into megakaryocytes by the general methods, an induction gene may be introduced into cells so that the induction gene into megakaryocytes is expressed; or a specific nucleic acid, protein, low molecular weight compound, or the like may be added to a culture solution to differentiate, into megakaryocytes, the cells that do not a capacity to be differentiated into megakaryocytes by the general methods. Therefore, it is possible to differentiate the cells into megakaryocytes.

<Incorporation step of incorporating magnetic fine particles into at least megakaryocytes>

As magnetic fine particles, any of fine particles of a superparamagnetic material, fine particles of a paramagnetic material, and fine particles of a ferromagnetic material may be used, but fine particles of a superparamagnetic material are preferably used.

Paramagnetism refers to magnetism that has no magnetization in a case where there is no external magnetic field, and that has weak magnetization in a direction of a magnetic field being applied.

Ferromagnetism refers to magnetism of a material in which adjacent spins are aligned in the same direction and has a large magnetic moment as a whole. A ferromagnetic material can have spontaneous magnetization without an external magnetic field.

Superparamagnetism is a property that is expressed in nanoparticles of a ferromagnetic material. In magnetic nanoparticles, a direction of magnetization may be reversed randomly due to the influence of temperature, and time until the reversal occurs is called the Neel relaxation time. In a state where there is no external field, when measurement time of magnetization of magnetic nanoparticles is much longer than the Neel relaxation time, the magnetization appears to be zero on average, which is a state called superparamagnetism.

An average particle diameter of the magnetic fine particles used in the present invention is not particularly limited, but is preferably 10 to 1000 nm, is more preferably 10 to 500 nm, and is even more preferably 10 to 100 nm.

An average particle diameter of the magnetic fine particles can be measured by photon correlation spectroscopy.

Examples of fine particles of a superparamagnetic material include fine particles containing iron (Fe), manganese (Mn), cobalt (Co), nickel (Ni), zinc (Zn), or gadolinium (Gd), and particles containing iron (Fe) are preferable.

Preferable examples of fine particles containing iron (Fe) include superparamagnetic iron oxide (SPIO) fine particles. Specific examples thereof include particles containing iron oxide (II) (FeO), iron oxide (II, III) ($Fe_3O_4$), iron oxide (III) ($\gamma$-$Fe_2O_3$), or mixtures thereof.

Superparamagnetic iron oxide is preferably coated with a polymer. Superparamagnetic iron oxide coated with carboxydextran (iron oxide (III) ($\gamma$-$Fe_2O_3$)) (for example, Resovist (registered trademark), and superparamagnetic iron oxide coated with dextran (a mixture of iron oxide (II) (FeO) and iron oxides (II, III) ($Fe_3O_4$) (for example, Feridex (registered trademark)) are more preferable.

Examples of the incorporation step of incorporating magnetic fine particles into at least megakaryocytes include physical methods such as an incubation method in which magnetic particles are added to a culture solution containing megakaryocytes (or megakaryocytes and platelets), electroporation, microinjection, sonoporation, and laser irradiation; and chemical methods such as transfection.

Among the methods, preferably, it is possible to adopt the incubation method in which magnetic fine particles are added to a culture solution containing megakaryocytes (or megakaryocytes and platelets). In the incubation method, magnetic fine particles are added to a culture solution containing megakaryocytes (or megakaryocytes and platelets), and incubated at a suitable temperature (for example, 25°C to 50°C, preferably 30°C to 45°C) for suitable time (for example, 1 hour to 48 hours, preferably 4 hours to 24 hours).

In a case where magnetic fine particles are added to a culture solution containing megakaryocytes (or megakaryocytes and platelets), a concentration of the magnetic fine particles is not particularly limited as long as the object of the present invention can be achieved. The concentration thereof is preferably 1 to 1000 $\mu$g/ml, is more preferably 10 to 1000 $\mu$g/ml, is even more preferably 100 to 1000 $\mu$g/ml, and is particularly preferably 500 to 1000 $\mu$g/ml.

Incorporation of the magnetic fine particles into the cytoplasm of megakaryocytes (or megakaryocytes and platelets) can be checked by preparing a stain sample of cell sections of cultured cells and staining them with an appropriate stain solution such as Berlin Blue.

<Culture step of culturing megakaryocytes in culture solution to produce platelets>

In the present invention, a culture step of culturing the megakaryocytes in a culture solution to produce platelets is performed before and/or after the incorporation step. In other words, in the present invention, separation of megakaryocyte and platelets may be applied with respect to a mixed solution of megakaryocytes and platelets after megakaryocytes are cultured to produce platelets in advance, or may be applied with respect to a mixed solution obtained by producing platelets after incorporating magnetic fine particles into megakaryocytes.

Accordingly, the present invention may be any of the following aspects.

(Aspect 1) Megakaryocytes are cultured in a culture solution to produce platelets, magnetic fine particles are incorporated into the megakaryocytes and the platelets, a magnetic field is applied to the culture solution, and then the culture solution is recovered.

(Aspect 2) Magnetic fine particles are incorporated into megakaryocytes, the megakaryocytes are cultured in a culture solution to produce platelets, a magnetic field is applied to the culture solution, and then the culture solution is recovered.

(Aspect 3) Megakaryocytes are cultured in a culture solution to produce platelets, magnetic fine particles are incorporated into the megakaryocytes and the platelets, the megakaryocytes are further cultured in the culture solution to produce platelets, a magnetic field is applied to the culture solution, and then the culture solution is recovered.

Among the above aspects, the aspect 1 or the aspect 2 is preferable, and the aspect 1 is more preferable.

The culture step of culturing megakaryocytes in a culture solution to produce platelets can be performed according to general methods for culturing cells. A culture period is not particularly limited, but is generally 3 hours to 30 days, is preferably 24 hours to 20 days, and is more preferably about 3 days to 14 days.

<Magnetic field application step of applying magnetic field to culture solution>

Examples of means (magnetic field application means) for applying a magnetic field to a culture solution include a method using a permanent magnet or an electromagnet. Examples of permanent magnets include ceramic magnets, metal magnets, and bond magnets, and ceramic magnets and metal magnets are preferable. Examples of ceramic magnets or metal magnets include samarium cobalt magnets, alnico magnets, ferrite magnets, and neodymium magnets. A samarium cobalt magnet is a magnet that has samarium and cobalt as main components. An alnico magnet is a magnet cast by using aluminum, nickel, cobalt, or the like as a raw material. A ferrite magnet is a magnet formed of a ferrite magnetic material. A neodymium magnet is a magnet that has neodymium, iron, and boron as main components.

The above-mentioned permanent magnets may be used alone, or a device including a permanent magnet may be used. An electromagnet may be a superconducting electromagnet.

In the magnetic field application step, a magnetic field of 10 to 400 mT is preferably applied to a culture solution, and a magnetic field of 10 to 150 mT is more preferably applied to a culture solution. Time for applying a magnetic field is not particularly limited, but is generally 30 seconds to 5 minutes.

<Recovery step of recovering culture solution after magnetic field application step>

More magnetic fine particles are expected to be incorporated into megakaryocytes than platelets produced from the megakaryocytes. Accordingly, by applying a magnetic field to a culture solution containing megakaryocytes and platelets, a large number of megakaryocytes move along a magnetic field direction as compared to platelets. Therefore, platelets can be efficiently recovered by recovering a culture solution after the magnetic field application step.

The movement of megakaryocytes into which magnetic fine particles have been incorporated along the magnetic field direction can be checked by observing cells with a microscope or the like.

In order to check an abundance (the number of cells) of megakaryocytes and platelets in a recovered solution, the recovered solution can be reacted with a labeled anti-CD41a antibody and then reacted with Hoechst 33342 which is a nuclear stain agent, and flow cytometry can be performed. Megakaryocyte fractions and platelet fractions are determined, and CD41-positive cells and nuclear stain negative cells in the platelet fractions are perceived as platelets, and CD41-positive cells and nuclear stain positive cells in the megakaryocyte fractions are perceived as megakaryocytes. Therefore, the number of platelets and the number of megakaryocytes in the recovered solution can be calculated.

<Platelet separation kit>

According to the present invention, a platelet separation kit for separating megakaryocytes and platelets which includes at least a culture vessel; magnetic fine particles; and magnetic field application means, is provided.

The culture vessel is not particularly limited as long as it is a vessel suitable for culturing cells and applying a magnetic field to the cells. For example, it is possible to use a plate, a dish, a petri dish, or a culture flask. As the plate, a multiwell plate (for example, a 6-well plate, a 24-well plate, and the like) may be used.

As magnetic fine particles and magnetic field application means, it is possible to use the magnetic fine particles and the magnetic field application means which have been described above in the present specification.

The present invention will be described more specifically by the following examples, but the present invention is not limited by the examples.

The following abbreviations have the following meanings.

FBS: Fetal bovine serum

DPBS: Dulbecco's Phosphate-Buffered Saline

PE: Phycoerythrin

7-AAD: 7-amino-7- actinomycin D

Examples

<Example 1>

(1) Preparation of cell fluid

StemSpan SFEM II (manufactured by STEMCELL Technologies Inc.) into which StemSpan megakaryocyte expansion supplement (manufactured by STEMCELL Technologies Inc.) and Penicillin-Streptomycin Solution ($\times$ 100) (manufactured by Wako Pure Chemical Industries) were added was used as a differentiation-inducing medium for preparing human megakaryocytes and platelets. By using the above-described differentiation-inducing medium, BM-CD34$^+$ Stem/Progenitor Cells (Allcell) were cultured for 14 days on a low adherent 6-well polystyrene plate (Ultra-Low Attachment Surface, manufactured by Corning Incorporated). The floating cells in culture were recovered in a 15 mL tube (manufactured by Corning Incorporated) and centrifuged at 1700 × g for 5 minutes at room temperature. The supernatant was discarded and the cells were suspended in DPBS (100 μl) containing 1% FBS (manufactured by Thermo Fisher Scientific). 10 μl of PerCP-Cy5.5 (registered trademark)-labeled anti-CD41a antibody (manufactured by Nippon Becton, Dickinson and Company) and 10 μl of PE-labeled anti-CD42b antibody (manufactured by Nippon Becton, Dickinson and Company) were added, and reaction was performed for 30 minutes with light shielding. DPBS (1 mL) containing 1% FBS was added, the mixture was centrifuged at 1700 × g for 5 minutes at room temperature, and the supernatant was discarded. The cells were suspended in DPBS (100 μl) containing 1% FBS to which 7-AAD was added, reacted for 15 minutes with light shielding, DPBS (300 μl) containing 1% FBS was added thereto, and measurement was performed by flow cytometry (manufactured by Becton, Dickinson and Company (BD), FACS Aria). Megakaryocyte fractions and platelet fractions were determined from a forward-scattered light (FSC) by side-scattered light (SSC) gate with use of MEG-01 (purchased from American Type Culture Collection (ATCC)), which is a megakaryocyte cell line, as a control. The presence of CD41a-positive cells and CD42b-positive cells was checked by comparison of each antibody with a sample stained with isotype control antibodies, and therefore megakaryocyte differentiation from BM-CD34$^+$ Stem/Progenitor Cells and platelet production from the megakaryocytes were confirmed.

(2) Introduction of magnetic fine particle into cells

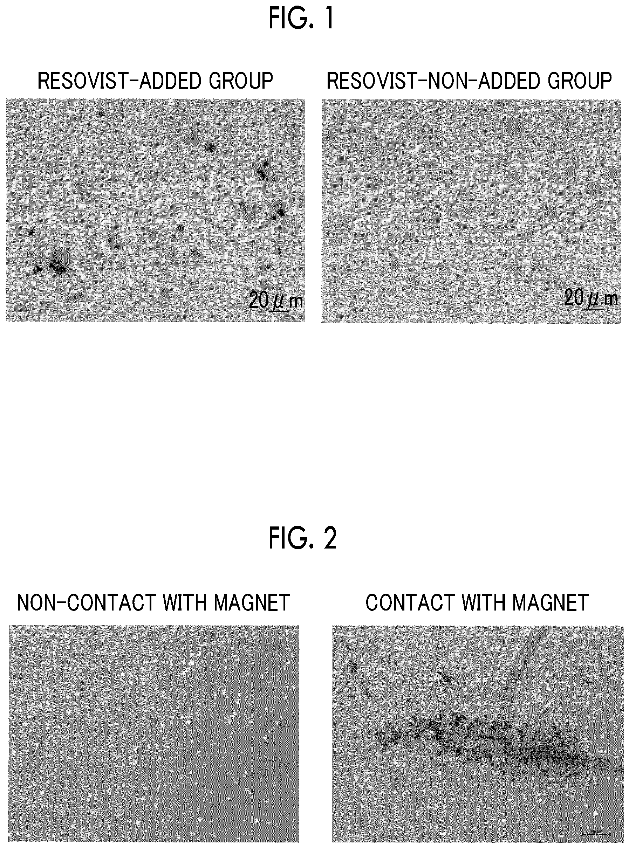

Per 1 mL of culture solution, 3.59 μL (100 μg of superparamagnetic iron oxide) of Resovist (registered trademark) injection (Fujifilm RI Pharma), which is a hydrophilic colloid liquid and which is formed of superparamagnetic iron oxide with an average particle diameter (photon correlation spectroscopy) of about 57 nm which is coated with carboxydextran, was added to the human megakaryocyte and platelet culture solution obtained by the method of (1) described above, and incubated at 37°C for 16 hours. In order to confirm that superparamagnetic iron oxide had been incorporated into the cytoplasm of megakaryocytes, cultured cells were embedded in paraffin, and then sliced to a thickness of 5 μm by using a microtome to prepare a stain sample of cell sections, and results of performing berlin blue staining (manufactured by Wako Pure Chemical Industries, Ltd.) are shown in Fig. 1.

(3) Magnetic field application to magnetized cells

By bringing a cylindrical-shaped samarium cobalt magnet with a diameter of φ 20 × 10 (mm) into contact with the bottom surface of the plate of the culture solution obtained by the method (2) described above for 1 minute, it was confirmed that megakaryocytes into which superparamagnetic iron oxide was incorporated were accumulated on the center portion of the plate (Fig. 2). In order to recover megakaryocytes and platelets not accumulated on the magnet, the entire supernatant of the culture solution was recovered in a 15 mL conical tube for centrifugation (manufactured by Falcon) which contains a citrate dextrose solution (ACD) (manufactured by Sigma-Aldrich). In addition, washing was performed twice with DPBS (manufactured by Thermo Fisher Scientific), and a wash fluid was recovered to the conical tube.

(4) Counting the number of recovered cells

The recovered solution was recovered in a 15 mL tube and centrifuged at 1700 x g at room temperature for 10 minutes. The supernatant was discarded and the cells were suspended in DPBS (100 μl) containing 1% FBS. 10 μl of PerCP-Cy5.5 (registered trademark)-labeled anti-CD41a antibody was added and reacted for 30 minutes with light shielding. DPBS (1 mL) containing 1% FBS was added, and the mixture was centrifuged at 1700 × g for 10 minutes at room temperature, and the supernatant was discarded. The cells were suspended in DPBS (100 μL) containing 1% FBS into which Hoechst 33342 (manufactured by Dojin Chemical Research Institute), which is a nuclear stain agent, was added, and reacted for 15 minutes in a light-shielding environment. DPBS (300 μL) containing 1% FBS was added thereto, and measurement was performed by flow cytometry (FACS Aria) by using BD Trucount tubes (manufactured by Nippon Becton, Dickinson and Company). Megakaryocyte fractions and platelet fractions were determined from a FSC by SSC gate with use of MEG-01 which is a megakaryocyte cell line as a control. CD41-positive cells and nuclear stain negative cells in the platelet fractions are perceived as platelets, and CD41-positive cells and nuclear stain positive cells in the megakaryocyte fractions are perceived as megakaryocytes. Therefore, the number of platelets and the number of megakaryocytes in the recovered solution was calculated. A recovery percentage of platelets and a recovery percentage of megakaryocytes obtained from the following equation are shown in Table 1.

Recovery percentage of platelets (%) = (the number of platelets in recovered solution obtained after applying magnetic field/the number of platelets in original solution) × 100

Recovery percentage of megakaryocytes (%) = (the number of megakaryocytes in recovered solution obtained after applying magnetic field/the number of megakaryocytes in original solution) × 100

Removal percentage of megakaryocytes (%) = 100 - recovery percentage of megakaryocytes (%)

A separation performance of platelets obtained by applying a magnetic field after incorporating superparamagnetic iron oxide was classified based on the following index.

Index value (I): recovery percentage of platelets (%)/recovery percentage of megakaryocytes (%)
Index value (II): removal percentage of megakaryocytes (%)
Separation performance A: index value (I) ≥ 1.5, index value (II) ≥ 25%
Separation performance B: index value (I) < 1.5, index value (II) ≥ 25%
Separation performance C: index value (I) < 1.5, index value (II) < 25%

<Examples 2 to 6 and Comparative Example 1>

The same operation as in Example 1 was carried out except that, per mL of a culture solution, 0.0359 μL (1.00 μg, Example 2), 0.359 μL (10.0 μg, Example 3), 1.79 μL (50.0 μg, Example 4), 17.9 μL (500 μg, Example 5), and 35.9 μL (1000 μg, Example 6) of Resovist (registered trademark) injection was added to a culture solution of human megakaryocytes and platelets in the method of (2) of Example 1. For Comparative Example 1, the same operation as in Example 1 was carried out without the addition of Resovist. The results are shown in Fig. 3 and Table 1.

<Examples 7 to 9 and Comparative Example 2>

The same operation as in Example 1 was carried out except that a magnet to be brought into contact with the bottom surface of a plate was changed to a cylindrical-shaped alnico magnet with a diameter of φ 20 × 10 (mm) (Example 7), a ferrite magnet (Example 8), or a neodymium magnet (Example 9) in the method of (3) of Example 1. A magnetic flux density of a magnetic field applied to the culture solution in a case where each magnet was brought into contact with the bottom surface was measured by using a Teslameter (TM-701, manufactured by Kanetec Co., LTD.).
In Comparative Example 2, the same operation as in Example 1 was performed without applying a magnetic field. The results are shown in Table 2.

[Table 1]

| | Amount of superparamagnetic iron oxide added ($\mu$g/mL) | Recovery percentage of platelets (%) | Recovery percentage of megakaryocytes (%) | Removal percentage of megakaryocytes (%) | Recovery percentage of platelets (%)/recovery percentage of megakaryocytes (%) | Separation performance |
|---|---|---|---|---|---|---|
| Example 1 | 1.00E + 02 | 28.3% | 21.6% | 78.4% | 1.3 | B |
| Example 2 | 1.00E + 00 | 80.1% | 59.0% | 41.0% | 1.4 | B |
| Example 3 | 1.00E + 01 | 42.4% | 49.3% | 50.7% | 0.9 | B |
| Example 4 | 5.00E + 01 | 41.7% | 37.1% | 62.9% | 1.1 | B |
| Example 5 | 5.00E + 02 | 36.0% | 18.4% | 81.6% | 2.0 | A |
| Example 6 | 1.00E + 03 | 25.2% | 13.2% | 86.8% | 1.9 | A |
| Comparative Example 1 | 0.00E + 00 | 90.6% | 96.3% | 3.7% | 0.9 | C |

[Table 2]

|  | Magnet | Magnetic flux density (mT) | Recovery percentage of platelets (%) | Recovery percentage of megakaryocytes (%) | Removal percentage of megakaryocytes (%) | Recovery percentage of platelets (%)/recovery percentage of megakaryocytes (%) | Separation performance |
|---|---|---|---|---|---|---|---|
| Example 1 | Samarium cobalt | 180.0 | 28.3% | 21.6% | 78.4% | 1.3 | B |
| Example 7 | Alnico | 18.2 | 82.6% | 34.2% | 65.8% | 2.4 | A |
| Example 8 | Ferrite | 100.9 | 47.5% | 26.6% | 73.4% | 1.8 | A |
| Example 9 | Neodymium | 351.2 | 15.4% | 25.6% | 74.4% | 0.6 | B |
| Comparative Example 2 | - | 0.0 | 110.4% | 1038% | -3.8% | 1.1 | C |

**Claims**

1.  A method for separating megakaryocytes and platelets, the method comprising:

    an incorporation step of incorporating magnetic fine particles into at least megakaryocytes;
    a culture step of culturing the megakaryocytes in a culture solution to produce platelets before and/or after the incorporation step;
    a magnetic field application step of applying a magnetic field to the culture solution after the incorporation step and the culture step; and
    a recovery step of recovering the culture solution after the magnetic field application step.

2.  The method according to claim 1, wherein the megakaryocytes are cultured in the culture solution to produce the platelets, the magnetic fine particles are incorporated into the megakaryocytes and the platelets, a magnetic field is applied to the culture solution, and then the culture solution is recovered.

3.  The method according to claim 1, wherein the magnetic fine particles are incorporated into the megakaryocytes, the megakaryocytes are cultured in the culture solution to produce platelets, a magnetic field is applied to the culture solution, and then the culture solution is recovered.

4.  The method according to any one of claims 1 to 3, wherein the magnetic fine particles are fine particles having an average particle diameter of 10 to 1000 nm.

5.  The method according to any one of claims 1 to 4, wherein the magnetic fine particles are superparamagnetic iron oxide particles.

6.  The method according to any one of claims 1 to 5, wherein 1 to 1000 $\mu$g/ml concentration of the magnetic fine particles are added to the culture solution in the incorporation step.

7.  The method according to any one of claims 1 to 6, wherein 10 to 400 mT of a magnetic field is applied to the culture solution in the magnetic field application step.

8.  A platelet separation kit for separating megakaryocytes and platelets, the kit comprising, at least:

    a culture vessel;
    magnetic fine particles; and
    magnetic field application means.

# FIG. 1

RESOVIST-ADDED GROUP

RESOVIST-NON-ADDED GROUP

20 μm

20 μm

# FIG. 2

NON-CONTACT WITH MAGNET

CONTACT WITH MAGNET

## INTERNATIONAL SEARCH REPORT

| | International application No. |
|---|---|
| | PCT/JP2018/010507 |

**A. CLASSIFICATION OF SUBJECT MATTER**
Int.Cl. C12N5/078(2010.01)i, C12M1/00(2006.01)i

According to International Patent Classification (IPC) or to both national classification and IPC

**B. FIELDS SEARCHED**

Minimum documentation searched (classification system followed by classification symbols)
Int.Cl. C12N5/078, C12M1/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JMEDPlus/JST7580 (JDreamIII),
CAplus/MEDLINE/EMBASE/BIOSIS/WPIDS/WPIX (STN)

**C. DOCUMENTS CONSIDERED TO BE RELEVANT**

| Category* | Citation of document, with indication, where appropriate, of the relevant passages | Relevant to claim No. |
|---|---|---|
| Y<br>A | JP 2006-06166 A (THE UNIVERSITY OF TOKYO) 12 January 2006, claims 1-37, paragraphs [0007]-[0010], [0025]-[0028] & WO 2006/001196 A1 | 8<br>1-7 |
| Y<br>A | JP 11-335289 A (ASAHI MEDICAL CO., LTD.) 07 December 1999, claim 1, paragraph [0005] (Family: none) | 8<br>1-7 |
| Y<br>A | JP 2016-190824 A (TORAY INDUSTRIES, INC.) 10 November 2016, claim 1 (Family: none) | 8<br>1-7 |
| Y<br>A | JP 2016-193896 A (TORAY INDUSTRIES, INC.) 17 November 2016, claim 1 (Family: none) | 8<br>1-7 |

☒ Further documents are listed in the continuation of Box C.    ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 June 2018 (11.06.2018) | 19 June 2018 (19.06.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2018/010507

C (Continuation).   DOCUMENTS CONSIDERED TO BE RELEVANT

| Category* | Citation of document, with indication, where appropriate, of the relevant passages | Relevant to claim No. |
|---|---|---|
| Y<br>A | BRAYLAN, Raul, et al., "Rapid and Simple Procedure For Isolation and Concentration Of Human Megakaryocytes From Marrow Aspirates", Blood, 2013, vol. 122, no. 21, p. 5269, see the whole document | 8<br>1-7 |
| Y<br>A | WO 2014/208100 A1 (KEIO GIJUKU) 31 December 2014, claim 1 & US 2016/0177265 A, claim 1 | 8<br>1-7 |
| A | JP 2007-089563 A (HITACHI METALS, LTD.) 12 April 2007, claim 1 (Family: none) | 1-8 |
| A | JP 6-133784 A (RES DEV CORP OF JAPAN) 17 May 1994, claim 1 (Family: none) | 1-8 |
| A | JP 2008-228728 A (UNIV. WASEDA) 02 October 2008, claims 1-4 (Family: none) | 1-8 |

Form PCT/ISA/210 (continuation of second sheet) (January 2015)

**EP 3 597 732 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 09122747 A
- JP 2003093499 A
- JP 2006006166 A
- WO 2004110139 A